# EUROPEAN PATENT APPLICATION

(11) **EP 2 537 923 A1**
(43) Date of publication of application: **26.12.2012**
(21) Application number: 11170788.1
(22) Date of filing: 21.06.2011
(51) Int. Cl.: C12N 5/0783

(54) **Method for activation of specific peripheral blood mononuclear cells**

(71) Applicant: Oncotyrol Center for Personalized Cancer Medicine GmbH, 6020 Innsbruck (AT)
(72) Inventor: Thurnher, Martin, 6071 Aldrans (AT); Grünbacher, Georg, 39030 Pfalzen Südtirol (IT); Nussbaumer, Oliver, 6020 Innsbruck (AT)
(74) Representative: Sonn & Partner Patentanwälte

(57) **Abstract**

The invention discloses a method for the preparation of stimulated cytotoxic (αβ and γδ) T cells and NK cells, comprising the following steps:
- providing a preparation containing CD1b-positive (CD1b+) antigen presenting cells and responder lymphocytes (T cells and NK cells),
- stimulating the preparation of CD1b+ antigen presenting cells and responder lymphocytes with an anti-CD1b antibody and interleukin 2 (IL-2), and
- cultivating the stimulated CD1b+ antigen presenting cells and responder lymphocytes in a culture medium to obtain stimulated cytotoxic (αβ and γδ) T cells and NK cells.

## Description

The present invention relates to the activation of specific peripheral blood mononuclear cells.

The innate immune system, which includes many different cell types such as monocytes, granulocytes, natural killer cells, dendritic cells and γδ T lymphocytes provides immediate protection against a pathogen and initiates adaptive immune responses. T lymphocytes (T cells) and B lymphocytes (B cells) represent the cells of the adaptive immune system. In contrast to innate lymphocytes, they express clonotypic receptors (T cell receptors and B-cell receptors) to specifically recognize particular antigens. During adaptive immune responses, which are promoted by the innate immune system, T cells with appropriate T cell receptors specific for potentially infectious or dangerous antigens are activated and expanded to be available in large numbers as effector cells for the immediate combat against the infectious or dangerous agent (microbial or tumour cells). While most of the effector cells subsequently disappear, some of them persist to become memory T cells, which provide protection during re-encounter with the infectious or dangerous agent.

The CD4-positive T helper (Th) cells are critical mediators of the cellular immune response against pathogens and tumour cells. For many years, based on cytokine expression profiles, CD4-positive Th cell differentiation has been considered to occur along a dichotomy of lineages referred to as Th1 and Th2. Type 1 cytokines include IL-2, IFN-γ, IL-12 and TNF-ß, while type 2 cytokines include IL-4, IL-5, IL-6, IL-10 and IL-13. However, Th cell research during the last decade has demonstrated that the picture is more complex. A unique subset of Th cells, which is required for protection against extracellular pathogens including fungi, was shown to produce IL-17A, IL-17F, IL-22 as well as TNF. IL-17 mediates recruitment of neutrophils and macrophages to infected tissues. Defining the cytokines (IL-1, IL-6, TGF-ß, IL-21, IL-23) that mediate differentiation of naive Th cells into IL-17-producing Th cells, both in vitro and in vivo, resulted in the establishment of the Th17 cell subset as a separate Th cell lineage. Aberrant regulation of Th17 cells may play a significant role in the pathogenesis of multiple disorders including inflammatory and autoimmune diseases and possibly also cancer.

Passive immunotherapy by adoptive transfer versus active immunotherapy by vaccination: Adoptive immunotherapy represents a treatment that passively supports the immune system in order to fight diseases, such as cancer and infections. For this purpose, lymphocytes including T cells and NK cells are collected from a patient and expanded by cell proliferation in the laboratory. This increases the number of lymphocytes that are able to kill cancer cells or fight infections. These lymphocytes are administered back to the patient to increase the immune system's capacity to fight the disease.

Active immunotherapy, in contrast, attempts to stimulate the host's intrinsic immune response to an infectious disease or cancer. While non-specific active immunotherapy is designed to generally boost the immune system using, for instance, cytokines, specific active immunotherapy (vaccination) attempts the generation of cell-mediated and antibody immune responses directed against specific antigens expressed by the pathogen or cancer cells.

A vaccine is a biological composition that is designed to enhance immunity to a particular disease. Typically, a vaccine consists of an agent that resembles a disease-causing pathogen or tumour cell. The agent stimulates the patient's immune system to recognize the agent as dangerous, destroy it, and build up immunologic memory, so that the immune system can more easily recognize and destroy any of these threats upon re-encounter.

Vaccines can either be prophylactic to prevent or ameliorate the effects of a future infection or therapeutic (vaccines against cancer). The efficacy of a vaccine can be further enhanced by an adjuvant, which can be a pharmacological or immunological agent that improves the effect of the vaccine.

Heat shock proteins (HSP) are a class of functionally related proteins whose expression is increased, when cells are exposed to elevated temperatures or other stress. Extracellular and membrane bound heat-shock proteins, especially Hsp70 are involved in binding antigens and presenting them to the immune system and have thus adjuvant properties.

Dendritic cells: Dendritic cells (DCs) are the professional antigen presenting cells (APCs) that initiate and regulate a broad repertoire of immune responses (Steinman et al., Nature (2007), 419-426). DCs link innate and adaptive immunity. In addition to their elaborate capability to activate peptide antigen-specific T cells in a T cell receptor (TCR) dependent and MHC-restricted fashion, DCs can also activate glycolipid antigen-specific T or NKT cells in a TCR-dependent but CD1-restricted manner (De Libero et al., Nat. Rev. Immunol. 5 (2005), 485-96). Moreover, DCs interact with and activate γδ T cells as well as natural killer (NK) cells, which are important effector cells in innate immune responses against pathogens and tumours.

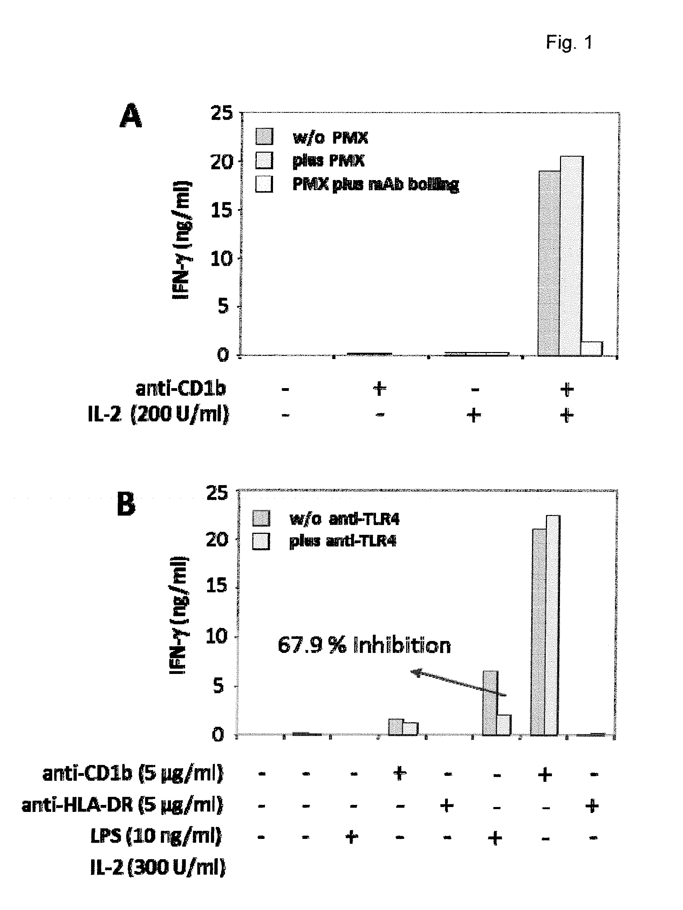

Dendritic cells in immunotherapy: DCs are ideally suited for the induction of immune responses that protect from infectious diseases or cancer. As outlined in Figure 1, an immunotherapy protocol consists of the following steps:
a) the isolation of precursor cells from patient blood using, for instance, immunomagnetic techniques;
b) the differentiation and final maturation of the DCs;
c) the loading of the differentiating/maturing DCs with (tumour) antigen;
d) the (repeated) administration of the antigen-loaded, mature DCs to the patient.

The current limitations of DC-based immunotherapy include the limited efficacy of antigen loading, a lack of physiological maturation protocols that do not exhaust DCs, the limited capacity of DCs to migrate in vivo as well as the fact that the protocol is time consuming and expensive.

The human family of CD1 antigens is expressed on DCs: The human CD1 protein family currently consists of three groups (De Libero et al., Nat. Rev. Immunol. 5 (2005), 485-96). Based on their sequence similarity CD1a, CDlb and CD1c are classified as group 1 CD1 proteins (present in humans but not in mice). Group 2 consists of CD1d only and CDle again represents a separate group. These evolutionarily conserved, MHC class I-like CD1 proteins comprise a family of antigen-presenting molecules that are capable of presenting nonpeptide lipid and glycolipid antigens to T cells. It is well established that these CD1 proteins are expressed on DCs present in both lymphoid and non-lymphoid tissues. Many of the well-characterized bacterial lipid antigens derived from the cell wall of Mycobacterium tuberculosis are presented on CD1b. Likewise, CD1b+ DCs have been demonstrated to play an important role in infectious diseases and can activate CD1b-restricted T cells with similar glycolipid specificity. Resting monocytes lack group 1 CD1 expression but can be induced to express CD1 proteins during activation in response to GM-CSF suggesting that CD1 proteins are up-regulated, when monocytes differentiate toward DCs in inflammatory lesions. Moreover, after toll-like receptor (TLR) activation of monocytes, endogenous GM-CSF promotes the differentiation of CD1b+ CD209 (DC-SIGN)-negative DCs.

The group I CD1 proteins in humans were first identified as differentiation markers expressed on immature cortical thymocytes and many monoclonal antibodies (mAb) directed against CD1 antigens have been generated by using thymocytes as an immunogen.

In human thymus, expression of CD1a, CD1b, and CD1c molecules has been reported to be developmentally regulated (Haynes et al., J. Exp. Med. 181 (1995), 1445-58). Analysis of human thymocyte phenotype at the time of initial colonization of the human thymus at 8.2-wk revealed asynchronous expression of CD1a, CD1b, and CDlc molecules. At this time only CDlb was expressed by all thymocytes, whereas CD1c was present on only 1% of 8.2-wk thymocytes and CD1a could only be detected on rare scattered macrophage-like cells. By week 12.75, however, CD1a, -b, and -c expression was similar to that seen in postnatal thymus, with the typical CD1 pattern of all cortex and few medullary cells being CD1⁺.

In addition to thymocytes, CD1 is also expressed by leukemic lymphoblasts of T cell lineage from peripheral blood of T-ALL patients and cell lines derived from such cells (MOLT-4, Jurkat). Down-regulation of CD1 marks acquisition of functional maturation of human thymocytes. So far, CD1 expression could not be detected on normal, non-malignant peripheral T lymphocytes.

Innate lymphocytes: natural killer (NK) cells: Natural killer (NK) cells are innate immune lymphocytes that mediate 2 major functions: recognition and lysis of tumour and virus-infected cells and production of immunoregulatory cytokines. The activation of an NK cell to kill a target cell is controlled by a complex interaction between activating and inhibitory receptor signals and can be modulated by cytokines. The production of cytokines such as interferon (IFN)-γ by NK cells is critical to early host defence against a variety of viral, bacterial, and parasitic pathogens. Innate immune signals that stimulate NK cell cytokine production are well established and include monokines such as interleukin 12 (IL-12), IL-15, IL-18, and IL-1ß and NK receptor ligation. DCs can also activate NK cells. The reciprocal impact of this interaction includes NK cell-mediated DC differentiation and maturation as well as DC-mediated NK cell activation.

Human NK cells comprise approximately 10% of peripheral blood lymphocytes and are characterized phenotypically by the presence of CD56 and the lack of CD3 (Caligiuri, Blood 112 (2007), 461-469). The majority (approximately 90%) of human NK cells are CD56^{dim} and express high levels of FcγRIII (CD16), whereas a minority (approximately 10%) are CD56^{bright} and CD16^{dim/neg}. CD56^{bright} NK cells constitutively express the high affinity IL-2 receptor and low affinity IL-2 receptor and expand in vitro and in vivo in response to low doses of IL-2 but do not themselves make IL-2.

γδ T lymphocytes: γδ T cells, which represent less than 10% of human peripheral blood T cells, can be classified according to their expression of TCR variable (V) region segments into Vδ1 T cells and Vδ2 T cells, which also differ in their tissue distribution and their antigen specificity (Kabelitz et al., Cancer Res. 67 (2007), 5-8). In the blood of most healthy individuals T cells expressing Vδ2 paired with Vγ9 account for 50% to >90% of all γδ T cells. Vδ2 T cells recognize phosphoantigens such as the mevalonate pathway-derived isopentenyl pyrophosphate (IPP). IPP is increased in malignant cells and IPP accumulation can be triggered by pharmacological inhibition of the IPP-metabolizing enzyme farnesyl pyrophosphate synthase using bisphosphonates such as zoledronate or pamidronate. In contrast, Vδ1 T cells are considered tissue γδ T cells, which are present in intestinal epithelia, in the skin and may be abundant among tumour-infiltrating lymphocytes. Vδ1 T cells recognize MHC class I-chain-related molecule A and B as well as UL-16 binding proteins. Some of the Vδ1 T cells have been reported to recognize CD1c. Most γδ T cells lack CD4 and CD8 expression, which is in accordance with their MHC-non-restricted recognition of unconventional antigens. Effector function of γδ T cells involves strong cytotoxic activity and cytokine production. Although γδ T cells are predominantly Th1 and produce IFN-γ and TNF-α, Th2-type γδ T cells have also been described. Moreover, the importance of IL-17-producing γδ T cells in models of infectious disease, immune-mediated disease and in injury models is increasingly becoming apparent. γδ T cell effector function is controlled by both TCR-dependent and independent mechanisms including activating (NKG2D) and inhibitory receptors (CD94).

A novel and unexpected function of γδ T cells as APCs has recently also been described (Brandes et al., Science 309 (2005), 264-268). Short-term activation of γδ T cells with small molecular weight non-peptide phosphoantigens induces γδ T cell differentiation toward APCs (γδ T cell-APCs), which are very similar in phenotype and function to monocyte-derived DCs. Other stimuli than such small molecular weight non-peptide phosphoantigens, which can also promote the development of γδ T cell-APCs, have not been described.

Current methods to induce γδ T cell expansion: All Vγ9/Vδ2 T cells recognize a small microbial compound (E)-4-hydroxy-3-methyl-but-2-enyl pyrophosphate (HMB-PP), which is a natural intermediate of the non-mevalonate pathway of isopentenyl pyrophosphate (IPP) biosynthesis. HMB-PP is an essential metabolite in most pathogenic bacteria including Mycobacterium tuberculosis and malaria parasites, but is absent from the metabolism of the human host. HMB-PP/IL-2 co-administration either in vitro or in vivo has successfully been used to induce activation/expansion of Vγ9/Vδ2 T cells.

IPP itself is structurally related to HMB-PP and ubiquitous-ly present in all living cells including human cells, yet its potency in vitro is much lower. Of pharmacological interest and with bioactivities comparable to that of IPP are synthetic aminobisphosphonates such as zoledronate (Zometa) or pamidronate (Aredia) that are widely used to treat bone resorption. Increasing evidence suggests that these aminobisphosphonates are not recognized directly by Vγ9/Vδ2 T cells as an antigen but may act indirectly, via their effects on the mevalonate biosynthetic pathway, leading to an accumulation of the phosphoantigen IPP. Co-administration of either IPP or a bisphosphonate with IL-2 has also been used to expand Vγ9/Vδ2 T cells in vitro and in vi-vo in cancer patients.

Like all other T cells (αß T cells), γδ T cells can be activated and expanded by triggering the TCR-associated surface protein CD3 using a specific monoclonal antibody (signal 1) in combination with a co-stimulatory antibody directed against CD28 (signal 2). Such an antibody combination will lead to T cell expansion in the presence of the T cell growth factor IL-2. However, this method is nonspecific and will result in the activation of any T cell present in the initial cell population and will not induce the specific expansion of γδ T cells.

It is an object of the present invention to provide improved methods for activation of PBMCs, especially for the activation of innate and adaptive lymphocytes and for expansion of human T cells, especially γδ T cells, and NK cells.

Therefore, the present invention provides a method for providing a preparation of stimulated cytotoxic T cells and NK cells, comprising the following steps:
- providing a preparation of CD1b-positive (CD1b⁺) antigen presenting cells,
- stimulating the preparation of CD1b⁺ antigen presenting cells with an anti-CD1b antibody and interleukin 2 (IL-2), and
- cultivating the stimulated CD1b⁺ antigen presenting cells in a culture medium to obtain stimulated cytotoxic T cells and NK cells.

The present invention provides a novel method for the simultaneous in vitro (or in vivo) activation of innate and adaptive lymphocytes, which includes the expansion of human T cells, particularly γδ T cells, but also αβ T cells and NK cells by cell proliferation as well as the induction of effector functions (cytokine production and cytotoxicity). The invention also relates to the lymphocytes prepared by such a method and to their use in immunotherapy. Innate and adaptive lymphocytes can easily be prepared from peripheral blood or other sources of CD1b-positive cells and rapidly stimulated to produce important effector molecules and to exhibit potent cytotoxic activity against pathogens and tumour cells. Tumour cells killed by such activated lymphocytes in vitro represent a pharmaceutical composition meeting all requirements of a cancer vaccine, which can be loaded onto dendritic cells in vitro or in vivo. Using this method, T cells, particularly γδ T cells, and NK cells can be expanded to numbers sufficient to be used in adoptive immunotherapy approaches thus representing a pharmaceutical composition. Lymphocyte activation as induced by this method also results in the enhancement of, for instance, vaccine-induced peptide-specific immune responses, thus serving as a potent adjuvant (in a pharmaceutical composition) in approaches of antigen-specific vaccination. Lymphocyte activation and concomitant enhancement of protein-specific adaptive immune responses can be used in the prevention and treatment of infectious diseases or cancer. The method is also suitable to determine immune competence of a patient (diagnostic tool).

Collectively, the experimental evidence provided here proves that the versatile method according to the present invention is suitable for the enhancement of innate and adaptive immune responses. In particular, the invention relates to a method for the simultaneous and potent activation of NK and T cells, which are both major effector cells in the protection against pathogens and tumour cells. The invention further relates to the preparation of large numbers of activated T cells, particularly γδ T cells, which represent another subset of effector cells in the defence of pathogens and tumours. The method enables the expansion of γδ T cells by cell proliferation without the drawbacks of bisphosphonates which have intrinsic dose-limiting effects. The method of the present invention can also be used to kill tumour cells in vitro and generate a highly immunogenic tumour cell extract that by itself meets the requirements of a cancer vaccine, which can be administered to a cancer patient. Alternatively, the highly immunogenic tumour cell extract can be used for the simultaneous antigen-loading and maturation of a patient's dendritic cells in vitro. The antigen-loaded, non-exhausted, mature dendritic cells can then be administered as a tumour vaccine to the cancer patient. Finally, the method of invention can be used to enhance protein antigen-specific immune responses thus serving as a potent adjuvant of vaccine antigens.

The method according to the present invention comprises the stimulation of CD1b⁺ antigen presenting cells with an anti-CD1b antibody and IL-2. The cells obtained with the method according to the present invention show significant synergistic effects compared to cells which have been treated with anti-CD1b antibodies only (e.g. as disclosed in Theodorou et al., J. Immunol. 144 (1990), 2518-2523 or as disclosed in Taylor et al., J. Immunol. 147 (1991), 3794-3802): With the present method, not only conventional αβ T cells are activated, but also activation and expansion of γδ T cells takes place. Moreover, natural killer cells (NK cells) are activated. Besides the prototypic Th1 cytokin IFN-γ also Th17 cytokines are generated, especially IL-17A and IL-22. These synergistic effects lead to potent inflammatory responses by inducing the production of many other cytokines, chemokines and prostaglandins from many cell types including macrophages. The release of these cytokines attracts other cells including neutrophils. IL-17 function is also essential to a subset of CD4+ T cells therefore called T helper 17 (Th17) cells, which have been implicated in anti-tumour immunity (Aggarwal et al., J. Leukoc. Biol. 71 (2002), 1-8).

The CD1b⁺ antigen presenting cells can be any cell which is regarded as CD1b-positive cell (synonymous with "CD1b⁺ cells", "CD1b⁺ antigen presenting cells"), i.e. a cell which expresses CDlb and displays CDlb on the cell surface. One skilled in the art can use the procedures described herein, or known in the art, for determining whether a cell is CD1b⁺ or not. A preferred source of CD1b⁺ antigen presenting cells according to the present invention are peripheral blood mononuclear cells (PBMCs) or a subset thereof, especially a preparation of CD56⁺ PBMCs. Therefore it is preferred to provide preparation of CD56⁺ PBMCs as preparation of CD1b⁺ antigen presenting cells.

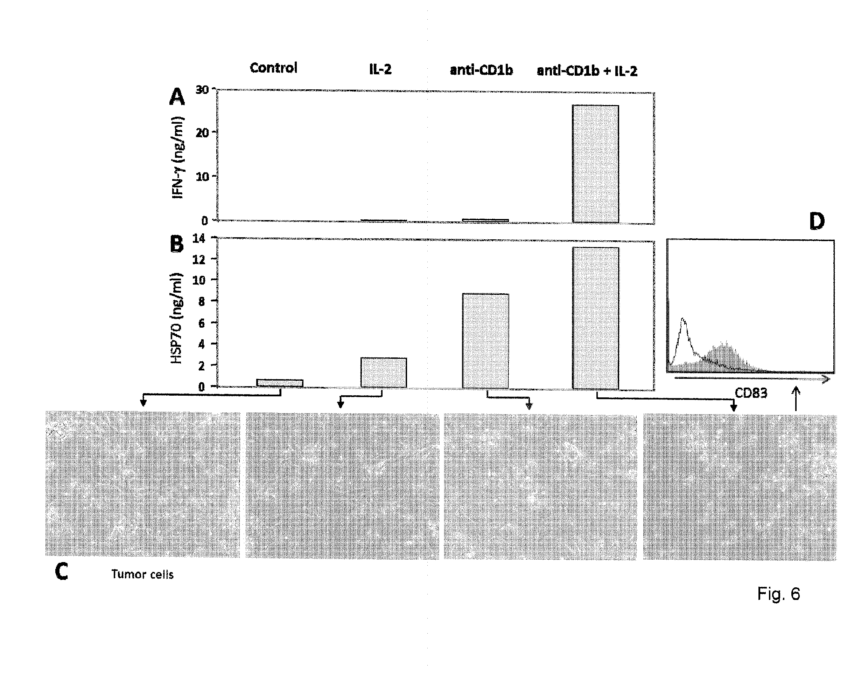

CD56⁺ PBMCs activated according to the present invention represent a preparation, which is enriched in highly cytotoxic lymphocytes with particularly strong antitumor activity (Fig. 6). Since the method according to the present invention enables the simultaneous activation of T cells and NK cells the final cell preparation comprises activated CD56⁺ γδ T cells, CD56⁺ αβ T cells as well as activated NK cells (which constitutively express CD56). Importantly, these subsets have all been reported to exhibit particularly strong cytotoxic activity (Alexander et al., Clin. Cancer Res. 14 (2008), 4232-4240; Pittet et al., J. Immunol. 164 (2000), 1148-1152; Caligiuri, Blood 112 (2007), 461-469).

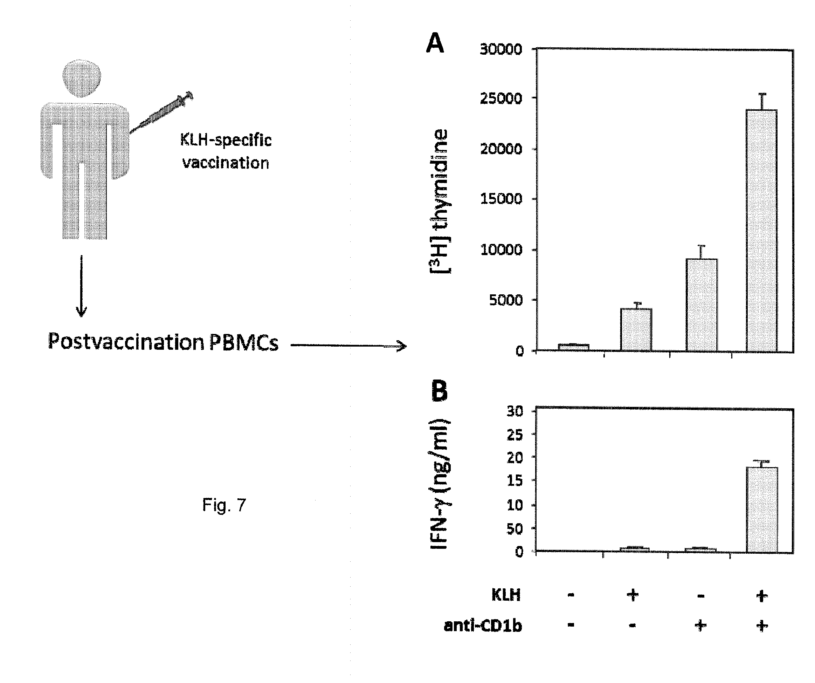

Preferably, PBMCs of healthy donors are provided, however, also PMBCs of patients, e.g. tumour patients, such as renal carcinoma patients can be used (see e.g. Fig. 7). However, in principle, any cell preparation containing CD1b⁺ antigen presenting cells can be used as starting material for the present method. For example, CD1b⁺ antigen presenting cells can be isolated from different tissue (e.g. epidermal cell preparations of dermatitis patients, such as in Taylor et al., 1991). Furthermore, e.g. cell preparations of lymphoid organs, such as spleen or lymph nodes can be used as well as tumour tissue. Nevertheless, the preferred source of CD1b⁺ cells is, as described above, mononuclear cells from human peripheral blood. Therefore, the preparation of CD1b⁺ antigen presenting cells is preferably obtained from peripheral blood, bone marrow or umbilical cord blood, especially peripheral blood. The method according to the present preparation may also be performed by provision of a preparation of lymphocytes or monocytes with a low level of CD1b⁺ antigen presenting cells or even without any CD1b⁺ antigen presenting cells being already present. Stimulation of this preparation with anti-CD1b antibody and IL-2 leads to the generation of CD1b⁺ antigen presenting cells which can then be cultivated. A "preparation of CD1b⁺ antigen presenting cells" therefore also includes a preparation comprising lymphocytes or monocytes which can be stimulated with an anti-CD1b antibody and IL-2 to become a preparation of CD1b⁺ antigen presenting cells during stimulation with anti-CD1b antibody and IL-2.

The stimulation of the CD1b⁺ antigen presenting cells according to the present invention is performed by the combined action of the anti-CD1b antibody and IL-2. This combination is much more effective in activation than the stimulation with anti-CD1b antibodies alone. IL-2 belongs to the family of common gamma chain cytokines, which also includes IL-4, IL-7 and IL-15. These cytokines were either much less effective or had no effect. However, IL-2 derivatives, such as pegylated IL-2 may be used, e.g. to achieve a depot effect. According to a preferred embodiment, IL-2 is added at a concentration of 5 to 5000 U/ml, preferably of 10 to 1000 U/ml, especially of 50 to 500 U/ml.

The stimulation according to the present invention is achieved by the addition of the combination of anti-CD1b antibodies and IL-2 and subsequent cultivation of the cells which allows the cell preparation to develop the novel phenotype and characteristics. In doing so, the cells are preferably cultured for a sufficient time at suitable conditions. According to a preferred embodiment of the present invention, cultivation is performed for at least 6, preferably for at least 12, especially for at least 16 hours. It is preferred to allow cultivation for about 1 to 3 days, however, also cultivation of at least one week or at least two weeks is possible. Cultivation of more than four weeks or even more than 6 or 8 weeks is not recommendable.

Preferred antibody concentrations for in vitro treatment of the cells are from 0.1 to 50 µg/ml, preferably from 0.5 to 20 µg/ml, especially from 1 to 10 µg/ml. Preferred IL-2 concentrations for in vitro treatment of the cells are 1 to 5000 U/ml, preferably 10 to 1000 U/ml, especially from 50 to 500 U/ml. Preferred temperatures for the in vitro cultivation are 30 to 40°C; preferred CO₂ concentrations are - as for all other parameters - the conditions optimised for the given cells and buffer systems, e.g. 1 to 10%, especially about 5% CO₂; for other buffer systems (such as HEPES), CO₂ concentrations could differ.

Accordingly, the preparation of CD1b⁺ antigen presenting cells preferably contains a cell culture medium which allows the cells to develop the desired properties according to the present invention.

After cultivation, the cell preparation obtained is preferably washed to remove the stimulating substances attached to the cells and from the cultivation solution and, optionally, also to remove cell products generated during cultivation of the stimulated cells. For some embodiments of the present invention (see below), it is, however, preferred to not remove the stimulating substances or cultivation products (such as cytokines) from the cell preparation, if such substances are needed in further steps (e.g. applying to a patient, stimulating immune cells, lysing tumour cells, etc.).

In a preferred embodiment (e.g. if the cell preparation is intended to be directly applied to a patient), the stimulated cytotoxic T cells and NK cells obtained after cultivation are washed with an isosmotic T cell washing solution, especially saline, phosphate buffered saline (PBS), or other neutrally buffered solutions, especially cell culture media.

The stimulated cytotoxic T cells and NK cells may then be finished to an injectable pharmaceutical formulation, especially an intradermally, intravenously, intraarterially or subcutaneously injectable formulation or a formulation to be injected into a lymph node (intranodally). A person skilled in the art is well aware of the ingredients, administration devices, packages, storage devices, etc. needed for the different administration routes.

The cultivation of the stimulated cells may be monitored in order to observe an appropriate point in time to end or interrupt cultivation. It is preferred to perform the cultivation at least for a duration until T cells and NK cells start to proliferate. This can easily be observed by measuring cell aggregate generation or cell density via optical microscopy. Usually, the cells start to proliferate after 1 to 3 days of culturing.

It is also possible to split the preparation containing the cultured stimulated cytotoxic T cells and NK cells during cultivation and preferably add culture medium and/or further cultivate the preparation. This results in an expansion of the T cells and NK cells in vitro, especially an expansion of stimulated cytotoxic γδ T cells.

Preferred culture media are AIM-V (Invitrogen), CellGro (CellGenix), RPMI1640 (Lonza) supplemented with 1-5 % autologous human plasma or human AB plasma.

According to a preferred embodiment, cultivation is performed for 6 h to 30 d, preferably from 12 h to 20 d, especially from 24 h to 10 d. Cultivation may be performed at any temperature at which cells can be stimulated and treated according to the present invention, e.g. at 30 to 40°C, preferably at 34 to 38°C, especially at 37°C.

One of the most important features of the method according to the present invention is the generation and expansion of γδ T cells. Therefore, the number of these cells may be specifically observed during cultivation. Accordingly, the cultivation may be performed for a time to increase the number of γδ T cells by a factor of at least 10, preferably of at least 50, especially at least 100.

The nature of the anti-CD1b antibody does not seem to be critical as long as it results in binding to the CD1b⁺ cells and allows the cells to take the developments according to the present invention, especially increasing the number of γδ T cells, secreting IFN-γ and Th17 cytokines, such as IL-17A and/or IL-22. Accordingly, the anti-CD1b antibody may be a CD1b-binding IgG, IgM, IgA, IgD or IgE antibody or a CD1b-binding fragment thereof, especially an Fab, F(ab')₂ or Fv; or an antibody derivative selected from CD1b-binding bi-, tri- or multispecific antibody, disulphide linked Fv, scFv, closed conformation multispecific antibody, disulphide-linked scFv, or diabody. As used herein an antibody refers to any antibody, antibody fragment or antibody derivative whether derived from any species naturally producing an antibody, or created by recombinant DNA technology; whether isolated from serum, B-cells, hybridomas, transfectomas, yeast, insect cells or bacteria. In general, one or more antibody variable domains can be incorporated to a given established antibody format or scaffold so as to confer binding specificity for antigen on the structure. A variety of suitable antibody formats are known in the art, such as, chimeric antibodies, humanized antibodies, human antibodies, single chain antibodies, bispecific antibodies, antibody heavy chains, antibody light chains, homodimers and heterodimers of antibody heavy chains and/or light chains, antigen-binding fragments of any of the foregoing (e.g. a Fv fragment (e.g., single chain Fv (scFv), a disulfide bonded Fv), an Fab fragment, an Fab' fragment, an F(ab')2 fragment), a single antibody variable domain (e.g. a dAb, VH, VHH, VL), and modified versions of any of the foregoing (e.g., modified by the covalent attachment of polyethylene glycol or other suitable polymer or a humanized VHH). The phrase "immunoglobulin single variable domain" refers to an antibody variable domain (VH, VHH, VL) that specifically binds an antigen or epitope independently of other V regions or domains. An immunoglobulin single variable domain can be present in a format (e.g., homo- or hetero-multimer) with other variable regions or variable domains where the other regions or domains are not required for antigen binding by the single immunoglobulin variable domain (i.e., where the immunoglobulin single variable domain binds antigen independently of the additional variable domains). A "domain antibody" or "dAb" is the same as an "immunoglobulin single variable domain" as the term is used herein. A "single immunoglobulin variable domain" is the same as an "immunoglobulin single variable domain" as the term is used herein. A "single antibody variable domain" or an "antibody single variable domain" is the same as an "immunoglobulin single variable domain" as the term is used herein. An immunoglobulin single variable domain is in one embodiment a human antibody variable domain, but also includes single antibody variable domains from other species such as rodent (for example, as disclosed in WO 00/29004, the contents of which are incorporated herein by reference in their entirety), nurse shark and Camelid VHH dAbs. Camelid VHH are immunoglobulin single variable domain polypeptides that are derived from species including camel, llama, alpaca, dromedary, and guanaco, which produce heavy chain antibodies naturally devoid of light chains. The VHH may be humanized.

One preferred anti-CD1b antibody to be used in the present invention is the B-B5 clone, which is an IgG1 antibody recognizing CDlb and CDlc (Dzionek et al., J. Immunol. 165 (2000), 6037-6046). Failure of antibodies recognizing CDlc only to stimulate such immune responses indicates that the specificity of the B-B5 antibody for CDlb is responsible for the functional activity of the B-B5 antibody. Human thymocytes or Jurkat T cells, which both express CD1b, were used as the immunogen in the generation of the B-B5 producing hybridoma clone. During early T cell development, thymocytes transiently express CD1a, CD1b, and CDlc antigens (Haynes et al., 1995). Adult T cells lack these antigens, however, they can re-express these CD1 antigens during malignant transformation. The T leukemic cell line Jurkat, for instance, expresses CD1a, CDlb and CD1c. This is why thymocytes and Jurkat T cells can be used as immunogens for CDlb antibody generation. The B-B5 antibody was assigned to the CD1 cluster during Workshop VII (MacDonald et al., In: Mason D, ed. Leucocyte Typing VII. Oxford, England: Oxford University Press; (2002), 315-319).

Another preferred anti-CD1b antibody to be used in the present invention is the WM25 clone (Favaloro et al., Immunol. Cell Biol. 65 (1987), 517-527; Favaloro et al., Disease Markers 4 (1986), 261-70), which is an IgG1 antibody recognizing CDlb only. Human thymocytes were used as the immunogen in the generation of the WM25 producing hybridoma clone. The WM25 antibody was assigned to the CD1 cluster during Workshop IV (Leucocyte Typing IV (1989). Oxford University Press).

The present invention also relates to preparation of stimulated cytotoxic T cells, including γδ T cells, and NK cells, obtainable by the present method. The cell preparation according to the present invention comprises specifically advantageous properties, such as the expression of IFN-γ and the Th17 cytokines, interleukin-17A (IL-17A) and/or interleukin-22 (IL-22).

The γδ T cells obtained by the present method are cytotoxic, i.e. they are able to directly kill tumour cells or co-stimulate the cytotoxicity of other cells such as NK cells against tumor cells (Maniar et al., Blood. 116 (2010), 1726-1733). Preferred cell preparations according to the present invention have a cytotoxicity of at least 10 % of all tumour cells, preferably at least 50 % of all tumour cells, especially at least 80 % of all tumour cells. It is, however, also possible to kill all or almost all tumour cells (100 %, 99 %, 98 %, 95 %, 90 %, etc.) by the method according to the present invention.

Although expansion of γδ T cells is primarily the aim of the present invention, the preparations obtained may also contain NK cells, αβ T cells, or mixtures of such cells. This also shows that the preparations according to the present invention are specifically advantageous, because known stimulants of γδ T cells (such as bisphosphonates) are not able to perform such additional stimulation.

The preparations according to the present invention may be finished to a form which can be pharmaceutically administered, e.g. by providing them in a pharmaceutically acceptable form, preferably by providing (or adding) an isosmotic T cell washing solution, especially saline, phosphate buffered saline (PBS), lactated Ringer's solution, or other clinically used carrier solutions or suspensions.

The pharmaceutical preparations according to the present invention may further contain added active substances (i.e. substances which are not (or not enough) expressed by the cells but actively added to the cell preparation). Preferred added substances comprise at least one tumour antigen, especially a preparation of lysed tumour cells; at least one cytokine, especially IL-2; at least one toll-like receptor agonist; a microbiological antigen or mixtures thereof.

The preparations according to the present invention preferably contain 1 to 10 x 10⁶ γδ T cells with antigen-presenting capacity or 1 x 10⁶ to 1 x 10⁸ γδ T cells as effector cells per dose. These preparations can be administered once or more to a patient in need thereof. If the present preparation is used as a vaccine, the preparation (dose) is administered preferably at least twice to the patient. Preferably, such vaccination may be performed each 1 to 4 weeks, e.g. for a duration of at least two, at least four or at least six months. If used as effector cells, the preparation can be administered e.g. each two months or each three months for at least six months or at least twelve months.

The preparation according to the present invention may also be stored, e.g. if a given patient should receive more than one administration of the cell preparation at different points in time, or transported. Accordingly, the preparation is preferably stored (or transported) in frozen form, e.g. by using 10% DMSO, 40% serum and 50% RPMI1640 Medium. It is also possible to lyophilise such cells (e.g. for vaccination purposes).

According to another aspect, the present invention relates to a method for the preparation of a tumour vaccine comprising the following steps:
- providing a preparation according to the present invention containing stimulated cytotoxic T cells and NK cells,
- contacting said stimulated cytotoxic T cells and NK cells with tumour cells, especially autologous tumour cells of a cancer patient,
- cultivating the mixture of stimulated cytotoxic T cells, NK cells and tumour cells to obtain a mixture comprising lysed tumour cells and pro-inflammatory cytokines, and
- optionally finishing the mixture of lysed tumour cells and pro-inflammatory cytokines to a pharmaceutical preparation.

Due to the advantageous properties of the cell preparations provided with the present invention, this method provides a very efficient tumour vaccine. Accordingly, the preparation according to the present invention then resembles not only a general immune stimulant but a tumour vaccine being specific against a certain tumour combined with a very effective adjuvant (i.e. the cells, cytokines and mediators obtained by the present invention). In the preparation of such a tumour vaccine, it is preferred to kill the tumour cells used in the present method or at least inhibit the tumour cells in growth so that they are still intact but do not grow anymore. Therefore, the tumour cells are preferably inhibited in growth, preferably by irradiation, chemotherapy drugs or combinations thereof.

According to a preferred embodiment of this method, the mixture comprising lysed tumour cells and pro-inflammatory cytokines is contacted for loading and/or maturation of dendritic cells (DCs) in vitro to obtain a DC-based tumour vaccine. The present tumour vaccine can be used for virtually any tumour disease, especially the most frequent solid tumours, especially colorectal cancer, esophagus cancer, gastrointestinal cancer, gallbladder cancer, lung cancer, breast cancer, oral cancer, ovarian cancer, liver cancer, pancreas cancer, kidney cancer, rectal cancer, stomach cancer, head and neck cancer, cancer of the nervous system, retinal cancer, non-small cell lung cancer, brain cancer, soft tissue cancer, lymphnode cancer, cancer of the endocrine glands, bone cancer, cervix cancer, prostate cancer or skin cancer; or a haematological tumour, myelodysplastic syndrome, preferably acquired aplastic anaemia, acute myeloid leukaemia, acute lymphatic leukaemia, Hodgkin lymphoma, non-Hodgkin lymphoma or multiple myeloma.

The present invention can also be used for providing a vaccine against infectious diseases, especially against a viral, bacterial, fungal or eukaryotic pathogen. The preparation according to the present invention can be activated by a microbiological antigen, such as the pathogen itself, by an inactivated preparation of the pathogen or by a specific antigen of the pathogen. All active vaccine concepts known in the art can easily be transformed to the present format according to the present invention. Accordingly, preferred vaccines according to the present invention are therefore vaccines against infections with Yellow Fever virus, Cytomegalovirus, Herpes Simplex, Herpes Zoster, Eastern Equine Encephalitis virus, Hepatitis A, B or C virus, Hepatitis Delta virus, Dengue virus, and Human Immunodeficiency virus 1 and 2, or more generally, African Swine Fever Virus, Arbovirus, Adenoviridae, Arenaviridae, Arterivirus, Astroviridae, Baculoviridae, Bimaviridae, Bimaviridae, Bunyaviridae, Caliciviridae, Caulimoviridae, Circoviridae, Coronaviridae, Cystoviridae, Dengue, EBV, HIV, Deltaviridae, Filviridae, Filoviridae, Flaviviridae, Hepadnaviridae (Hepatitis), Herpesviridae, Iridoviridae, Mononegavirus (e.g., Paramyxoviridae, Morbi-Uivirus, Rhabdoviridae), Myoviridae, Orthomyxoviridae (e.g., Influenza A, Influenza B, and parainfluenza), Papiloma virus, Papovaviridae, Paramyxoviridae, Prions, Parvoviridae, Phycodnaviridae, Picomaviridae (e.g. Rhinovirus, Poliovirus), Poxviridae (such as Smallpox or Vaccinia), Potyviridae, Reoviridae (e.g., Rotavirus), Retroviridae, Rhabdoviridae, Tectiviridae, Togaviridae (e.g., Rubivirus), herpes, pox, papilloma, corona, influenza, hepatitis, sendai, sindbis, vaccinia viruses, west nile, hanta, viruses which cause the common cold; infections with E. faecalis, L. innocua, S. aureus, M. luteus, S. epidermidis, S. pneumoniae, P. aeruginosa, M. hominis or any other bacterial or microbiological pathogen disclosed e.g. in WO 2011/060199 A, and any combination thereof.

According to another aspect, the present invention relates to a method for in vitro stimulation of white blood cells characterised in by the following steps:
- providing a preparation of white blood cells,
- contacting the preparation of white blood cells with an anti-CD1b antibody and interleukin 2 (IL-2), and
- cultivating the stimulated white blood cells, and
- measuring at least one phenotypic property of the stimulated and cultivated white blood cells.

This method can be used for diagnostic purposes for the in vitro analysis of blood or other samples from humans, especially human patients (e.g. human patients which are or are planned to be treated with the preparations according to the present invention.

Preferred phenotypic properties to be measured in this method are expression levels of one or more cytokines, preferably members of the Th1 (IFN-γ, TNF-α, IL-12), Th2 (IL-4, IL-5, IL-13) or Th17 cytokine family (IL-17A-F, IL-21, IL-22, IL-23) as well as homeostatic cytokines (IL-7, IL-15) or regulatory cytokines (TGF-β, IL-10). Phenotypic analysis also includes the detection and quantification of cellular subsets (dendritic cell subsets, monocyte/macrophage subsets, T cell subsets (including regulatory T cells), NK cell subsets, granulocyte subsets). Expansion of, for instance γδ T cells, is determined by assessing the frequency (in %) of T cells expressing a γδ T cell receptor in the starting population and in the population expanded according to the present invention, which is harvested at the end of the culture period. By considering frequency of γδ T cells and total cell numbers and by comparing starting and expanded population the expansion factor can be calculated.

This method is specifically suited for monitoring the immune status of patients. Therefore, the preparation of white blood cells may be obtained from a patient whose immune status has to be monitored. The immune status of the patient may then be defined by comparison with reference values for the properties measured. In many cases, more than one sample of a patient is subjected to this method during monitoring, e.g. 2, 3, 4, 5, at least 10, at least 20, at least 50, or at least 100 samples. Usually, such different samples from one and the same patient have been taken at different points in time (e.g. before a treatment and after a treatment or in the course of a therapy). For example, the samples can be taken daily, weekly, monthly, etc.. It follows that according to a preferred embodiment of this method
- at least two preparations of white blood cells are obtained from a patient at different points in time,
- the same one or more phenotypic properties are measured in the at least two preparations of white blood cells, and
- the measured phenotypic properties of the at least two preparations are compared with each other to monitor the immune status of this patient.

In various clinical settings, it may be desirable to determine the immune status (immune competence) of a patient. For instance, immune competence of a patient is prerequisite for selection of this patient for an immunotherapy regimen since lack of immune competence would result in immunotherapy failure. Likewise, it is important to monitor the recovery of immune competence after immune compromising treatments such as chemo-, radiation or immunosuppressive therapy. For such a purpose, PBMCs may be prepared from peripheral blood of a patient at appropriate time points (before, during and after a certain intervention) and stimulated with anti-CD1b plus IL-2. Cytokine levels as assessed by ELISA or multiplexing technology, activation of individual cell subsets and expansion of, for instance, γδ T cells represents a measure of immune competence, which can be correlated with the dosing and duration of the treatment and/or the course of the disease (prognosis).

According to another aspect, the present invention relates to a kit for stimulation of white blood cells comprising
- an anti-CD1b-antibody, and
- IL-2.

For basic and applied research purposes, these immunologic test kits can be provided, which enable the activation and/or expansion of white blood cells. Anti-CD1b antibodies in combination with IL-2 are part of such test kits in order to induce the activation and/or expansion of NK cells as well as T cells including γδ T cells. In such an application, PBMCs prepared from any donor are stimulated with anti-CD1b antibody plus IL-2 followed by a particular read out that may be, but is not restricted to, a cytokine measurement, phenotypic analysis or cell expansion (see above).

According to another aspect, the present invention also relates to a pharmaceutical composition comprising an anti-CD1b-antibody, preferably for use in immune stimulation, especially for the treatment of infectious diseases or tumour diseases.

With such a pharmaceutical preparation, the method for stimulating cytotoxic T cells and NK cells of the present invention can be performed in vivo by administering the anti-CD1b antibody and optionally IL-2.

For vaccination purposes, the (humanized) CDlb antibody can be admixed to any vaccine antigen (either microbial or tumour-associated antigens). The final vaccine formulation consisting of the antigen preparation and the anti-CD1b antibody (adjuvans) can be administered, typically as an intradermal or subcutanteous injection. In situ the anti-CD1b antibody can bind to CD1b-expressing APCs and induce immune enhancement analogous to the in vitro embodiments described herein. This immune enhancement generates a stimulatory milieu and favours a potent immune response against the vaccine antigen.

This pharmaceutical composition comprising an anti-CD1b antibody and optionally IL-2 therefore preferably comprises a vaccine antigen, especially a vaccine antigen for vaccination against an infectious disease or a tumour vaccination antigen.

The present invention is further described by the following examples and the drawing figures, yet without being limited thereto.
Fig. 1: CDlb targeting induces strong IFN-γ responses in the presence of IL-2: exclusion of endotoxin and Fc effects. (A) PBMCs were stimulated for 4 days with anti-CD1b (WM25) in the presence or absence of IL-2 (200 U/ml). IFN-γ was determined in supernatants on day 4. Polymyxin B (PMB) was used at 60 U/ml to inhibit effects of potential endotoxin (LPS) contaminations of CDlb antibody preparations. Alternatively, CDlb antibody preparations were inactivated by boiling (100°C) for 20 min. (B) PBMCs were stimulated with B-B5 IgG1 anti-CD1b or an IgG1 control antibody that also binds to APCs (anti-HLA-DR), or with LPS in the presence or absence of IL-2. IFN-γ was determined in supernatants on day 4.

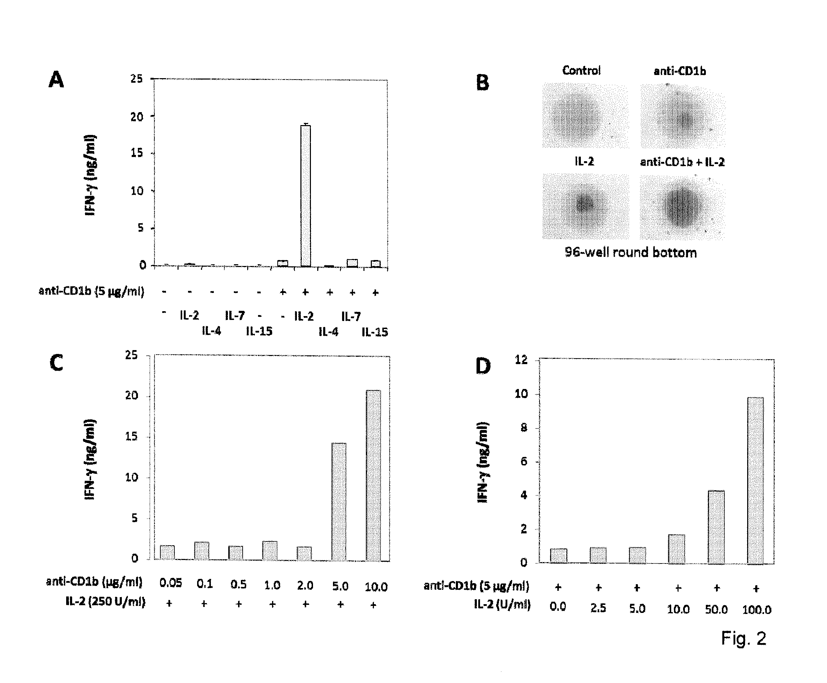

Fig. 2: CDlb targeting induces strong IFN-γ responses in the presence of IL-2: no effect of other homeostatic cytokines. (A) PBMCs were stimulated for 4 days with anti-CD1b (WM25) in the presence or absence of IL-2 (200 U/ml), IL-4 (200 U/ml), IL-7 (25 ng/ml) and IL-15 (25 ng/ml). IFN-γ was determined in supernatants on day 4. Results are mean values of triplicate measurements ± SD. (B) Microscopic examination of PBMC aggregation in response to anti-CD1b plus IL-2. Increasing cell aggregates are indicative of cell activation and proliferation. (C) PBMCs were stimulated with graded doses of anti-CD1b (B-B5) in the presence of a constant IL-2 concentration. (D) PBMCs were stimulated with graded doses of IL-2 in the presence of a constant anti-CD1b concentration. IFN-γ was determined in supernatants on day 4.

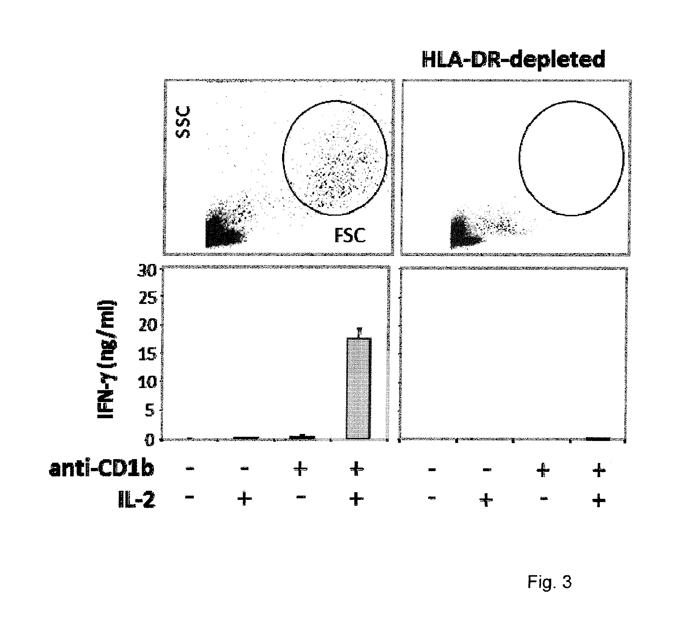

Fig. 3: IFN-γ production induced by anti-CD1b plus IL-2 depends on APCs. PBMCs (1 x 10⁶/ml) or PBMCs depleted of HLA-DR-positive cells were stimulated with IL-2 (100 U/ml), anti-CD1b (5 µg/ml) or anti-CD1b plus IL-2. IFN-γ was determined in supernatants on day 4. Forward scatter (FSC)/side scatter (SCC) analysis of cell preparations confirmed depletion of the APC population (top).

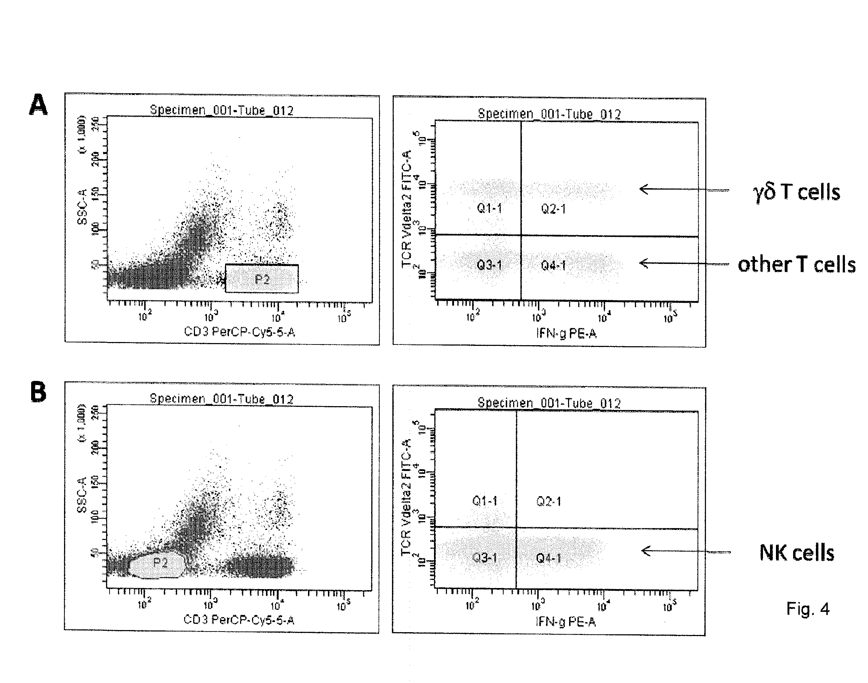

Fig. 4: T and NK cells produce IFN-γ in response to stimulation with anti-CD1b plus IL-2. CD56-positive PBMCs (1.5 x 10⁶/ml) were stimulated for 24 h with anti-CD1b (5 µg/ml) plus IL-2 (100 U/ml). Cells were stained for intracellular IFN-γ and for surface CD3 (all T cells) as well as TCR Vδ2 (γδ T cells only). (A) Gating on CD3-positive (all T cells); (B) Gating on CD3-negative cells (NK cells).

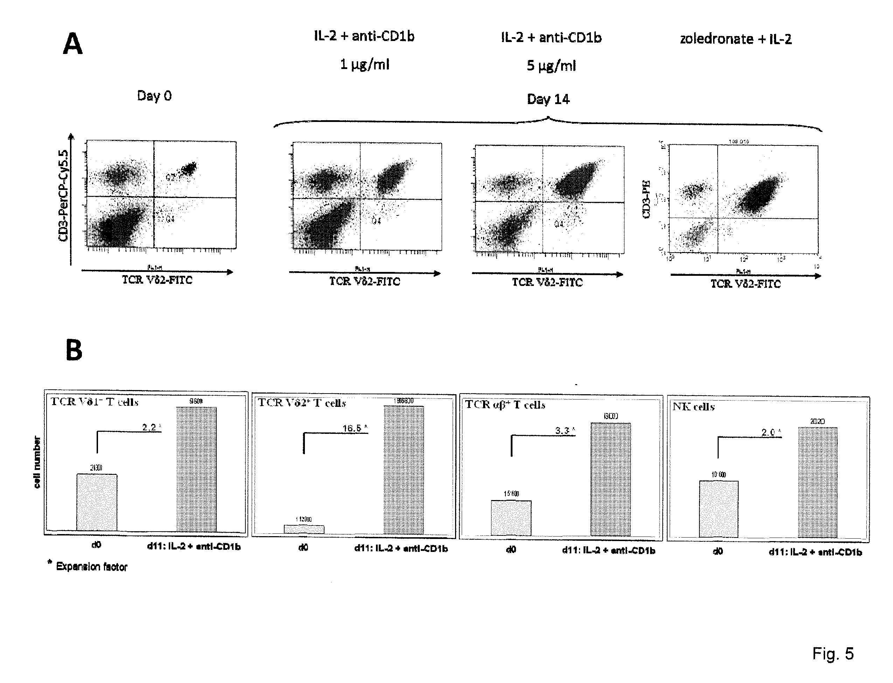

Fig. 5: Efficient lymphocyte expansion after stimulation with anti-CD1b and IL-2. (A) γδ T cell expansion: CD56-positive PBMCs (1.5 x 10⁶/ml) were stimulated with either IL-2 alone (100 U/ml), or with anti-CD1b (5 µg/ml) plus IL-2, or with zoledronate (1 µM) plus IL-2. Fresh complete medium containing IL-2 was added every 3 days. After 14 days of expansion, cells were harvested, counted and analyzed for TCR Vδ2+ T cell frequency by flow cytometry. Plots of CD3 expression against Vδ2 expression show TCR Vδ2+ T cells in freshly isolated CD56-positive PBMCs and reveal efficient expansion with anti-CD1b plus IL-2, or zoledronate plus IL-2 but not with IL-2 alone. (B) CD56-positive PBMCs were stimulated with anti-CD1b plus IL-2. After 14 days of expansion, T cell subsets and NK cells were analyzed by flow cytometry. By considering frequency of cell subsets and total cell numbers and by comparing starting and expanded population the expansion factor was calculated.
Fig. 6: Potent cytotoxicity of PBMCs stimulated with anti-CDlb and IL-2 against human cancer cells. PBMCs (1.5 x 10⁶/ml) were co-cultured with human renal cell carcinoma (RCC) cells (1.5 x 10⁴/ml) and were either left untreated or stimulated with IL-2 (100 U/ml) or anti-CD1b (5 µg/ml) alone or with a combination of anti-CD1b and IL-2. (A) IFN-γ was determined in supernatants on day 4. (B) HSP70 was determined in supernatants on day 4. (C) Microscopic examination was used to monitor the destruction of the human RCC monolayer. (D) Supernatants from cocultures treated with the combination of anti-CD1b and IL-2 were added to immature CD83-negative DCs and up-regulation of the maturation marker CD83 was determined by flow cytometry.
Fig. 7: Enhancement of immune responses against a vaccine antigen by targeting of CD1b. PBMCs were harvested from renal cell carcinoma patients after the third vaccination with autologous, antigen-loaded, mature CD83⁺ DCs (=postvaccination PBMCs). PBMCs were either left unstimulated (control) or stimulated for 4 days with the vaccine control antigen KLH (10 µg/ml), with anti-CD1b (WM25) alone or with combinations of KLH and anti-CD1b. (A) Proliferative responses were assessed as [3H] thymidine incorporation. (B) IFN-γ contents were determined in culture supernatants.

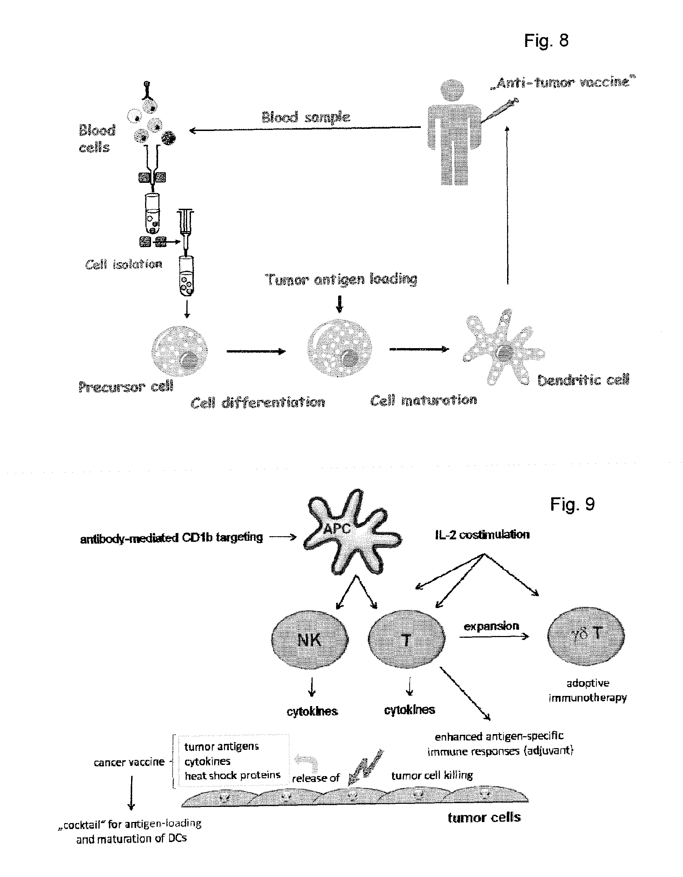

Fig. 8: Graphic representation of an immunotherapy protocol DC precursor cells are isolated from a patient's blood sample and differentiated into DCs using appropriate differentiation-inducing cytokines (e.g. GM-CSF and IL-4). After tumour antigen loading and a maturation step using e.g. inflammatory cytokines/mediators (TNF-α, IL-1ß, prostaglandin E2), the mature, antigen-loaded DCs are administered to the patient, which is frequently done by i.d. or i.n. injection. The (repeatedly) injected DCs are expected to induce antigen-specific immune responses directed against the patient's tumour.
Fig. 9: Graphic representation summarising important aspects of the present invention. The schematic presentation summarizes the most important aspects of the invention: In the presence of IL-2, antibody-mediated targeting of CDlb on APCs leads to APC-dependent activation of lymphocytes including T cells and NK cells. Both lymphocytes and APC produce multiple pro-inflammatory cytokines. Activated lymphocytes also exhibit potent cytotoxicity against tumour cells. As a consequence of tumor cell destruction ("lysis") tumour antigens and heat shock proteins are released. The resulting "cocktail" containing tumour antigens, pro-inflammatory cytokines and heat shock proteins meets all requirements of a cancer vaccine. The "cocktail" can also be used for the simultaneous antigen-loading and maturation of non-exhausted DCs. In the presence of IL-2, targeting of CDlb on APCs also leads to the expansion of T cells, particularly γδ T cells, as well as NK cells by cell proliferation. The expanded T cells and NK cells can be adoptively transferred to the patient, where they can exhibit potent antitumour activity. Targeting of CDlb can strongly enhance pre-existing antigen-specific immune responses, for instance, against a vaccine antigen, and thus serves as potent adjuvant. The measurement of proliferative, cytokine and cytotoxic responses after stimulation with anti-CD1b with or without IL-2 may be used to determine (diagnose) the immune status (immune competence) of healthy persons and patients.

### Examples

### Example 1: Cell isolation

Peripheral blood, bone marrow, umbilical cord blood may be typical sources of blood for PBMC preparation. Considering the advantages of accessibility and minimal invasiveness, the use of peripheral blood is preferable. It is preferable to collect a relatively small amount of blood that does not burden the donor. As a method of collection, whole blood can be collected utilizing a vacuum blood tube. Heparin or citric acid may be added to prevent blood coagulation. In cases where large numbers of cells are required, PBMCs can be directly obtained by use of a component collection system such as leukapheresis.

PBMCs are then separated from whole blood or from leukapheresis products. Any method for separating mononuclear cells may be used. For example, the Ficoll separation method, i.e., a Ficoll-Paque density gradient is frequently used. For this purpose LSM1077 Lymphocyte Separation Medium (PAA, Pasching, Austria) in combination with Leucosep (greiner bio-one, Frickenhausen, Germany) can be used for the optimal separation of PBMCs.

In order to remove platelets, it is preferable to wash the collected PBMCs several times by centrifugation using a culture medium, physiological saline, phosphate buffered saline (hereinafter referred to as PBS), which may further be supplemented with serum or albumin to prevent unspecific attachment of cells to plastic surfaces.

The CD56-positive subset may also be isolated from PBMCs, since this subset is enriched in NK cells as well as γδ T cells and also contains a population of DC-like APCs, which are suitable for the activation of T cells and NK cells (Gruenbacher et al., Blood 114 (2009), 4422-4431; Gruenbacher et al., Cancer Res. 70 (2010), 9611-9620; Nussbaumer et al. Blood (2011), in press). A method to isolate and collect CD56-positive cells utilizing the Magnetic Cell Sorting is preferred since it is simple and reliable and results in pure populations of CD56-positive PBMCs (>90%). In this method CD56 microbeads are used in combination with LS columns according to the manufacturer's instructions (MACS; Miltenyi Biotec, Bergisch-Gladbach, Germany).

### Example 2: Cell stimulation for NK cell and T cell activation

Antibodies against CD1 antigens, which are expressed by DCs, are used. Above all, antibodies against CDlb are particularly preferable. Examples include WM-25 IgG1 against CDlb and B-B5 IgG1 against CD1b/c (Favaloro et al., 1987; Favaloro et al., 1986; Dzionek et al., 2000).

The whole PBMCs or the CD56-positive PBMCs obtained are resuspended preferably at cell numbers of 1.0-1.5 x 10⁶/ml in a cell culture medium such as RPMI-1640 (Lonza, Basel, Switzerland), AIM-V (Invitrogen, Carlsbad, CA, USA) or CellGro SCGM (CellGenix, Freiburg, Germany). The cell culture medium may be further supplemented with autologous or clinical quality heterologous serum or plasma at 1-20%. There is no limitation for the culture vessels used, which may be a plate, laboratory dish, flask, bag typically used for cell cultivation.

An antibody directed against CDlb (clone B-B5 from Diaclone, Besancon, France or clone WM25 from ImmunoKontact via AMS Biotechnology, Lugano, Switzerland) is added to these cultures. The final concentration of the CDlb antibody in the cell culture to be stimulated is preferably 1-20 µg/ml. In addition, a T cell growth factor, preferentially interleukin-2 (IL-2, Proleukin, Novartis, Basel, Switzerland), is added to the cultures. An IL-2 concentration of 10-1000 U/ml can be used, with 100 U/ml being preferable. Some experiments were performed in the presence of polymyxin B (PMB from Sigma-Aldrich, Vienna, Austria; 10 µg/ml corresponding to 60 U/ml) to exclude effects of contaminating lipopolysaccharides (LPS). The B-F1 mouse IgG1 anti-HLA-DR (Diaclone, Besancon, France) was used as an isotype control. For neutralizing and blocking assays, cultures were pre-incubated with specific antibodies (5-10 µg/mL). The cultivation is carried out under the conditions of 34-38°C, more preferably 37°C, and 2-10% CO₂, more preferably 5% CO₂. After 1-5 days of incubation the cells or the culture supernatants were harvested for analysis or further assays. Activation as detected by the BD FastImmune (BD Biosciences, San Jose, CA, USA) of NK cells and T cells (both αß T cells and γδ T cells) occurs already within the first 12 to 16 hours. At this time point IFN-γ, which is a prototypic cytokine of effector cells and crucial for the defence of pathogens and tumours, can be detected intracellularly in T and NK cells and together with various other cytokines during the following days also in the supernatant using cytokine bead arrays (Th1/Th2 or Inflammation CBA; BD Biosciences) or the Human Th1/Th2/Th9/Th17/Th22 13plex Kit (eBiosciences, San Diego, CA, USA). The mixture of activated NK cells and T cells can itself be considered a pharmaceutical composition, which can be administered to a patient with cancer or an infectious disease for general immune enhancement. Alternatively, the mixture of activated NK cells and T cells can be further processed to become a) a cancer vaccine, b) a "cocktail" for the simultaneous antigen loading and maturation of dendritic cells in vitro, or c) a preparation of expanded T cells, particularly γδ T cells, and NK cells.

### Example 3: Generation of a cancer vaccine

Stimulation of PBMCs or subsets thereof is performed as described above in Example 2 (Cell stimulation for NK cell and T cell activation) and between day 0 and day 10 activated cells are seeded onto tumour cells growing in an appropriate cell culture vessel at a PBMC to tumour cell ratio of 10:1 to 1:1. The tumour cells may either be autologous, i.e. primary tumour cells derived from the patient to be treated, or represent a permanent cell line derived from another individual but of the same tumour type, i.e. allogeneic tumour cells. The tumour cells may be irradiated (¹³⁷Cs) at a dose of about 30 to 100 Gray, which leaves them intact but prevents growth of tumour cells potentially surviving the attack of the activated NK and T cells. The activated NK cells and T cells in the PBMC preparation will attack, kill and lyse the tumour cells. Tumour cell lysis is monitored by microscopic inspection and by measuring the release of tumour-associated peptides such as M30 or Hsp70 using specific ELISAs (M30 Apoptosense ELISA from PEVIVA AB, Bromma, Sweden; HSP70 ELISA from ENZO Life Sciences/Eubio, Vienna Austria). In addition, NK cell and T cell activation is monitored by measuring multiple cytokines at once using cytokine bead arrays (Th1/Th2 or Inflammation CBA; BD Biosciences) or Human Th1/Th2/Th9/Th17/Th22 13plex Kit (eBiosciences, San Diego, CA, USA). On day 5, the tumour cells will be destroyed completely and the resulting tumour cell extract ("cocktail"), which contains a representative mixture of tumour-associated antigens and variety of pro-inflammatory cytokines, can be administered to the cancer patient as a potent, immunogenic cancer vaccine. Preferentially, the "cocktail" will be divided into equal portions (i.e. aliquots), which can be stored frozen at -20°C to - 180°C for many months or even years and thawed for the repetitive administration (i.e. vaccination) of the patient. Administration occurs by intradermal or subcutaneous injection, or by injection into a lymph node adjacent to a lesion, or even by direct injection into a lesion.

### Example 4: Generation of an immunogenic tumour cell extract for simultaneous loading and maturation of DCs in vitro

In a variation of Example 3, the resulting "cocktail", which contains a representative mixture of tumour-associated antigens and variety of pro-inflammatory cytokines, can be used for simultaneous loading and maturation of DCs in vitro. In this setting, in vitro generated DCs at the immature stage (compare Fig. 8; Steinman et al., 2007) and at a cell density of about 0.5-1.0 x 10⁶ cells/ml are incubated with appropriate doses of the "cocktail", for instance, by removing half of the DC medium and replacing it by "cocktail". During the incubation with "cocktail", the DCs use their efficient endocytic mechanisms to pick up, process and present the tumour antigens contained in the "cocktail". In addition, the abundant pro-inflammatory cytokines contained in the "cocktail" will promote DC maturation, which can be confirmed by measuring the up-regulation of the reliable maturation marker CD83 (clone HB15e-PE, BD Biosciences) using flow cytometry (Gruenbacher et al., 2009). Mature, antigen-loaded DCs can then be administered as a DC-based tumour vaccine by intradermal or intranodal injection as described previously (Höltl et al., Clin. Can. Res. 8 (2002), 3369-3376).

### Example 5: Expansion T cells, particularly γδ T cells, in vitro

PBMCs are prepared from a donor as described in Example 1 and stimulated for 5 days as described in Example 2. Every 2 days, half of the cell culture medium is replaced by fresh medium containing IL-2. The activated cells will form aggregates and T cells, particularly γδ T cells, but also NK cells will start to proliferate. When cell densities extend about 2.0 x 10⁶/ml and medium turns yellow (usually on day 5), the cultures will be split. For this purpose, the cells of a culture well are resuspended and half of the suspension is transferred to a fresh well. Both wells are filled up to the original volume with fresh medium containing IL-2. Following this protocol, the cells can be expanded for 10 to 20 days. The numbers of γδ T cells as determined by multicolour flow cytometry (Gruenbacher et al., 2009) initially present in PBMC preparations will increase about 10-100-fold using this procedure.

The present invention enables the generation of a cell population containing activated γδ T cells in large quantities and in a simple fashion. These activated γδ T cells can directly be used in adoptive immunotherapy and can thus be regarded as a pharmaceutical composition. After washing the expanded cells, they can be administered to a patient with, for instance, cancer or an infectious disease. Any washing solution may be used as long as it is isosmotic and suitable for use as a pharmaceutical preparation. Considering the subsequent administration to a patient, the use of Ringer Lactate (Baxter, Deerfield, IL, USA), physiological saline, PBS or the like is preferable. In addition, a clinical grade cytokine such as IL-2 (Proleukin) may also be added.

The number of cells administered will be selected depending on the actual purpose. Typically, however, the number of cells is preferably 1-10 x 10⁶ cells/person, when γδ T cells serve as APCs and 1 x 10⁸ cells to 1 x 10¹⁰ cells/person, when γδ T cells serve as antitumour effector cells. γδ T cells can be administered by intravenous, intradermal or subcutaneous injection, injected into a lymph node adjacent to a lesion, directly injected into a lesion, given as an intravenous bolus or drip-fed for systemic administration (Höltl et al., 2002). The preparation can also be injected into an artery next to a lesion.

In vivo, the activated and expanded γδ T cells are expected to have strong therapeutic effects by exhibiting direct (cytotoxic) and indirect antitumour or anti-pathogen effects (by exhibiting immunomodulatory effects and APC function).

### Example 6: Use of anti-CD1b antibody for in vitro stimulation as part of an immunologic test kit

PBMCs are prepared from a donor as described in Example 1 and stimulated for 1-5 days as described in Example 2. For basic and applied research purposes, immunologic test kits are marketed, which enable the activation and/or expansion of white blood cells. Anti-CD1b antibodies alone or in combination with other stimuli such as cytokines may be part of such test kits in order to induce the activation and/or expansion of NK cells as well as T cells including γδ T cells. In such an application, PBMCs prepared from any donor (Example 1) would be stimulated with anti-CD1b antibody plus IL-2 (Example 2) followed by a particular read out that may be, but is not restricted to, a cytokine measurement (Example 2), phenotypic analysis or cell expansion (Example 5).

### Example 7: Targeting of CDlb in vivo

The CDlb antibody itself can be considered a pharmaceutical composition. In a humanized form the antibody can be administered to patient in vivo with or without a second stimulus such as clinical grade IL-2 (Proleukin). After administration, for instance, via intradermal injection or intravenous infusion, the antibody can bind to CD1b-expressing APCs in vivo and induce immune enhancement analogous to the in vitro examples described above (Examples 2 to 5). Such administration of anti-CD1b antibody will lead to the activation of NK and T cells including γδ T cells and thus in immune enhancement, which is desirable in the treatment of infectious diseases or cancer.

### Example 8: CDlb as an adjuvant of a vaccine

For vaccination purposes, the (humanized) CDlb antibody could be admixed to any vaccine antigen (either microbial or tumour-associated antigens). The final vaccine formulation consisting of the antigen preparation and the anti-CD1b antibody (adjuvans) would be administered, typically as an intradermal or subcutanteous injection. In situ the anti-CD1b antibody can bind to CD1b-expressing APCs and induce immune enhancement analogous to the in vitro examples described above (Examples 2 to 5). This immune enhancement will generate a stimulatory milieu and favour a potent immune response against the vaccine antigen.

### Example 9: Diagnosing the immune status/immune competence of a patient

PBMCs are prepared from a donor as described in Example 1 and stimulated for 1-5 days as described in Example 2. In various clinical settings, it may be desirable to determine the immune status (immune competence) of a patient. For instance, immune competence of a patient is prerequisite for selection of this patient for an immunotherapy regimen since lack of immune competence would result in immunotherapy failure. Likewise, it is important to monitor the recovery of immune competence after immune-compromising treatments such as chemo-, radiation or immunosuppressive therapy. For such a purpose, PBMCs are prepared from peripheral blood of a patient (Example 1) at appropriate time points (before, during and after a certain intervention) and stimulated with anti-CD1b plus IL-2 (Example 2). Cytokine levels as assessed by ELISA or multiplexing technology, activation of individual cell subsets and expansion of, for instance, γδ T cells (Example 5) represent a measure of immune competence, which can be correlated with the dosing and duration of the treatment and/or the course of the disease (prognosis).

### Results

Fig. 1 demonstrates that antibody-mediated targeting of CDlb cooperated with recombinant IL-2 in a highly synergistic fashion to induce the production of very large amounts of IFN-γ by human PBMCs. Antibodies against other CD1 antigens such as the anti-CD1a antibodies OKT-6 and HI149 or the CDlc specific antibodies M-T101, L161 failed to induce such cytokine responses in human PBMCs.

Theoretically, similar IFN-γ responses could be induced by endotoxin (LPS) present in antibody preparations. LPS concentrations of the CDlb antibody preparations (WM25, B-B5, SN13/K5-1B8) as determined by the Limulus Amebocyte Lysate (LAL) assay [Limulus Amebocyte Lysate QCL-1000, CAMBREX] were generally low (< 10 ng/ml at an antibody concentration of 10 µg/ml). LPS effects can be prevented by using the LPS-binding agent polymyxin B (PMB). Conversely, antibody effects can be abolished by boiling, since antibodies are heat-labile proteins, while LPS is heat-resistant. Data shown in Fig. 1A demonstrate that the stimulatory activity of the CDlb antibodies was not due to LPS contamination, since PMB at 10 µg/ml (corresponding to 60 U/ml) failed to abolish IFN-γ production. In contrast, boiling almost completely abrogated the IFN-γ response induced by CDlb antibodies (Fig. 1A) strongly suggesting that the effect was mediated by the antibodies. Moreover, LPS from Salmonella abortus equi at 10 µg/ml failed to reproduce the effects seen with the CDlb antibodies (Fig. 1B) further excluding a role of LPS in the observed effects.

IgG antibodies may not only generate effects by binding to their specific antigen but also via their Fc γ portion, which can trigger Fc γ receptors expressed on other cells. Octagam human IgG (Octapharma) was used in all experiments at 50 µg/ml to block Fc γ receptors. To further exclude non-specific Fc effects, an azide-free IgG1 anti-HLA-DR antibody was used as an isotype control antibody that also binds to APCs and can thus present its Fc proportion to Fc receptor-expressing cells. Fig. 1B demonstrates that anti-HLA-DR failed to reproduce the effects observed with anti-CD1b thus excluding the possibility that any IgG1 that binds to APCs can trigger such PBMC activation. Finally, an Fc γ receptor blocking antibody (anti-CD16) did not reduce the response induced by anti-CD1b antibodies unequivocally excluding such non-specific Fc γ effects.

Fig. 2 demonstrates that antibodies against CDlb (WM-25, B-B5, SN13/K5-1B8) induced IFN-γ production in cultures of human PBMCs. CDlb antibodies cooperated with IL-2 but not with other members of the common γ chain family of cytokines (IL-4, IL-7, IL-15) to produce very large amounts of IFN-γ (Fig. 2A). In 7 different donors, the combination of anti-CD1b and IL-2 induced high level IFN-γ production (32.6 ± 21.4 ng/ml; IL-2 controls: 135 ± 69 pg/ml; p=0.01) (Table 1). As shown in Table 2 the cytokine response was not restricted to IFN-γ but also included the production of IL-17, IL-22, TNF-α, IL-1ß and IL-6.

Microscopic examination of PBMC cultures in 96-well roundbottom culture wells revealed cell aggregation in response to treatment with anti-CD1b or IL-2 and also demonstrated synergistic effects of the combination of anti-CD1b and IL-2 (Fig. 2B).

Antibodies to CDlb stimulated IFN-γ responses by PBMCs in a dose-dependent fashion (Fig. 2C). Likewise, IL-2 co-stimulated the effects of anti-CD1b antibody in a dose-dependent manner (Fig. 2D).

Fig. 3 illustrates the role of APCs and thus confirms the specificity of anti-CD1b effects. Aside from early T cell development or malignant T cells, CDlb expression is restricted to APCs, which express high levels of HLA-DR (MHC class II) for presentation of peptide antigens. To further confirm the specificity of the effects mediated by the CDlb antibodies, all HLA-DR-positive cells were depleted using HLA-DR microbeads prior to stimulation with anti-CD1b antibody. Depletion of HLA-DR-positive cells eliminates all CD1b-positive cells and thus eliminates all potential target cells of the anti-CD1b antibodies. Fig. 3 clearly demonstrates that depletion of HLA-DR-positive cells almost completely abolishes the IFN-γ response usually induced by anti-CD1b plus IL-2 and thus demonstrates that anti-CD1b antibodies mediate their effect via CD1b-expressing APCs.

T cells and NK cells are major sources of IFN-γ. To identify the IFN-γ producing population cell subset depletion experiments were performed. Depletion of CD3-positive cells (T cells) from PBMCs did not reduce IFN-γ production in response to stimulation with anti-CD1b and IL-2 (Table 3). In contrast, sequential depletion of CD3-positive cells and CD56-positive cells (NK cells), however, abrogated IFN-γ production almost completely (Table 3) confirming that T and NK cells are the potential sources of IFN-γ in response to treatment with anti-CD1b plus IL-2. The concomitant activation of APCs as evidenced by mono-kine production (TNF-α, IL-1ß and IL-6) indicates that mutual activation of lymphocytes and monocytes/DCs occurs during CDlb targeting and IL-2 co-stimulation.

Fig. 4 reveals IFN-γ production in various lymphocyte subsets of human PBMCs after stimulation with anti-CD1b antibody in combination with IL-2. To identify the cellular sources of IFN-γ within PBMCs, intracellular staining of IFN-γ was performed after 24 h of stimulation with anti-CD1b antibody in combination with IL-2. Cells were counterstained for TCPVδ2 to identify γδ T cells and for CD3 to distinguish T cells (CD3+) from NK cells (CD3-). Fig. 4 convincingly shows that anti-CD1b plus IL-2 was very potent in inducing IFN-γ production in all these subsets (γδ T cells, other T cells, NK cells).

Fig. 5 demonstrates that treatment of human PBMCs with anti-CD1b antibody in combination with IL-2 induces massive expansion of γδ T cells. In freshly isolated PBMCs (day 0), 5% to 10% of all T cells were γδ T cells expressing a Vδ2 TCR, which is the TCR required for the recognition of bisphosphonate-induced phosphoantigens such as IPP. Treatment with IL-2 alone for 14 days failed to induce Vδ2-positive T cell expansion. However, after two weeks of anti-CD1b-induced and IL-2 supported expansion, Vδ2-positive T cells represented 50% to 70% of all cells (Fig. 5) actually resulting in an about 50 to 100-fold expansion, when the increase in absolute cell number was also considered. As expected, similar rates of γδ T cell expansion were achieved when zoledronate, which is the most potent bisphosphonate currently available, was combined with IL-2 (Fig. 5). To further assess the role of CD1b-positive APCs in γδ T cell expansion, replete PBMCs and PBMCs depleted of HLA-DR-positive cells were compared, i.e. all APCs including CD1b-positive cells, prior to stimulation with anti-CD1b antibody and IL-2. Depletion of HLA-DR-positive cells strongly reduced the efficiency of γδ T cell expansion.

At higher concentrations of bisphosphonates, γδ T cell expansion becomes less efficient, because protein isoprenylation is inhibited resulting in the attenuation of cell proliferation. With regard to bisphosphonates, it is a choice between γδ T cell proliferation (at lower concentration) and γδ T cell effector responses (IFN-γ production, cytotoxicity). In contrast, γδ T cell expansion (Fig. 5B) and IFN-γ production (Fig. 2A) both increase with anti-CD1b concentration representing a clear advantage of anti-CD1b as opposed to bisphosphonates.

Fig. 6 shows potent cytotoxicity of human PBMCs activated with anti-CD1b and IL-2 directed against human tumour cells. Then the cytotoxic antitumour effects of PBMCs activated with anti-CD1b plus IL-2 were studied. For this purpose, A-498 cells, a well-characterized example of human renal cell carcinoma (RCC), were cultured in 48-well plates. PBMCs were then added and stimulated with anti-CD1b and IL-2. Whereas the A-498 cell monolayer appeared relatively intact in control cultures or in cultures treated with either anti-CD1b or IL-2 alone, the combination of anti-CD1b plus IL-2 induced an almost complete destruction of the epithelial cell monolayer indicative of potent cytotoxicity (Fig. 6). Under conditions of combined stimulation with anti-CD1b and IL-2, not only high levels if IFN-γ were produced but also high levels of GM-CSF and IL-1ß as well as extraordinary high levels of IL-6 (Table 4). Moreover, tumour-associated antigens like heat shock protein (Hsp) 70, which has the properties of an immunological adjuvant, were released into the culture medium. The "conditioned" culture medium finally contained copious amounts of pro-inflammatory cytokines (Fig. 6A and Table 4), adjuvant heat shock proteins (Fig. 6B), as well as a variety of tumour antigens present in the tumour lysate (Fig. 6C) thus fulfilling all the requirements of a potent cancer vaccine. The "conditioned" culture medium can also be used to treat dendritic cells in vitro. Such a treatment serves two purposes at once: a) the tumour antigens present in the "conditioned" culture medium can be loaded onto dendritic cells also with the support of the endogenous heat shock proteins and b) the pro-inflammatory cytokines present in the "conditioned" culture medium will induce dendritic cell maturation, which is characterized by the up-regulation of the maturation marker CD83 without the drawbacks of DC exhaustion (Fig. 6D). As a consequence antigen-loaded, mature dendritic cells can be generated and subsequently administered to the tumour patient for the induction of antitumour immune responses in vivo.

Fig. 7 demonstrates that CDlb targeting can enhance antigen-specific, MHC-restricted T cell responses. PBMCs from renal cancer patients, who had been vaccinated with KLH-loaded DCs (Höltl et al., 2002), were used. Keyhole limpet hemocyanin (KLH) is a highly immunogenic protein derived from the giant keyhole limpet, Megathura crenulata, frequently used in vaccine research. The T cell response to KLH elicited by DC vaccination has previously been shown to be predominated by MHC class II-restricted CD4⁺ T cells. Postvaccination PBMCs were stimulated with KLH in the presence or absence of anti-CD1b antibody. KLH alone induced a proliferative response confirming KLH-specific immunity induced by DC vaccination. Likewise, CDlb targeting alone induced proliferative responses similar to those observed in healthy donors and anti-CD1b enhanced KLH-induced proliferation in a moderately synergistic fashion (Fig. 7A). CDlb targeting and KLH stimulation alone induced both low amounts of IFN-γ at comparable levels (Fig. 7B). The combination, however, of CDlb targeting and KLH stimulation resulted in a clearly synergistic enhancement of IFN-γ production (Fig. 7B) indicating that CDlb targeting can enhance the existing immunity against the vaccine antigen KLH.

**Table 1**

| | | **IFN-γ** *pg*/*ml* |
|---|---|---|
| Donor 1 | Control | 0,0 |
| | IL-2 | 67,0 |
| | anti-CD1b | 302,0 |
| | anti-CD1b + IL-2 | 32.466,0 |
| Donor 2 | Control | 2,0 |
| | IL-2 | 120,0 |
| | anti-CD1b | 606,0 |
| | anti-CD1b + IL-2 | 18.800,0 |
| Donor 3 | Control | 15,0 |
| | IL-2 | 228,0 |
| | anti-CD1b | 153,0 |
| | anti-CD1b + IL-2 | 28.517,0 |
| Donor 4 | Control | 19,0 |
| | IL-2 | 169,0 |
| | anti-CD1b | 826,0 |
| | anti-CD1b + IL-2 | 78.289,0 |
| Donor 5 | Control | 13,0 |
| | IL-2 | 356,0 |
| | anti-CD1b | 1.332,0 |
| | anti-CD1b + IL-2 | 83.859,0 |
| Donor 6 | Control | 17,0 |
| | IL-2 | 33,0 |
| | anti-CD1b | 992,0 |
| | anti-CD1b + IL-2 | 25.183,0 |
| Donor 7 | Control | 0,0 |
| | IL-2 | 194,7 |
| | anti-CD1b | 242,3 |
| | anti-CD1b + IL-2 | 12.508,5 |

**Table 2**

| | | **IFN-γ** *pg*/*ml* | **IL-17A** *pg*/*ml* |
|---|---|---|---|
| Donor 1 | IL-2 | 859,57 | 0 |
| | IL-2 + anti-CD1b | 6394,44 | 262,88 |
| Donor 2 | IL-2 | 521,99 | 10,56 |
| | IL-2 + anti-CD1b | 4427,6 | 1038,59 |

| | | **IL-22** *pg*/*ml* | **IL-6** *pg*/*ml* |
|---|---|---|---|
| Donor 1 | IL-2 | 219,62 | 135,84 |
| | IL-2 + anti-CD1b | 1259,11 | 7188,8 |
| Donor 2 | IL-2 | 389,72 | 83,19 |
| | IL-2 + anti-CD1b | 6745,64 | 10862,57 |

| | | **IL-1ß** *pg*/*ml* | **TNF-α** *pg*/*ml* |
|---|---|---|---|
| Donor 1 | IL-2 | 0 | 293,4 |
| | IL-2 + anti-CD1b | 2711,52 | 6995,68 |
| Donor 2 | IL-2 | 4,42 | 1263,14 |
| | IL-2 + anti-CD1b | 2521,65 | 4500,64 |

**Table 3**

| | | **IFN-γ** | **TNF-α** | **IL-6** | **IL-1ß** |
|---|---|---|---|---|---|
| | | *pg*/*ml* | *pg*/*ml* | *pg*/*ml* | *pg*/*ml* |
| PBMC | Control | 0,0 | 2,5 | 4,7 | 2,6 |
| | IL-2 | 194,7 | 23,3 | 18,9 | 1,9 |
| | anti-CD1 b | 242,3 | 42,8 | 3.558,6 | 86,7 |
| | IL-2 + anti-CD1 b | 12.508,5 | 880,1 | 9.816,1 | 444,0 |
| CD3⁻ PBMC | Control | 0,0 | 2,7 | 42,6 | 3,2 |
| | IL-2 | 224,0 | 9,0 | 67,9 | 4,1 |
| | anti-CD1b | 6,4 | 4,2 | 893,4 | 48,0 |
| | IL-2 + anti-CD1b | 20.095,7 | 5.368,8 | 32.039,9 | 3.094,2 |
| CD3⁻ CD56⁻ PBMC | Control | 0,5 | 2,2 | 70,7 | 5,2 |
| | IL-2 | 16,8 | 3,2 | 104,6 | 2,6 |
| | anti-CD1b | 2,5 | 2,3 | 367,4 | 24,2 |
| | IL-2 + anti-CD1b | 46,8 | 9,4 | 498,0 | 26,0 |

**Table 4**

| | | **IFN-γ pg/ml** | **GM-CSF pg/ml** | **TMF-α pg/ml** | **IL-6 pg/ml** | **IL-1ß pg/ml** |
|---|---|---|---|---|---|---|
| w/o tumor | Control | 11.0 | 26.5 | 18.6 | | 3.3 |
| | IL2 (250U/ml) | 1.050,4 | 2.186,4 | 77 2 | 34 5 | 54.9 |
| | anti-CD1b (5µg/ml) | 356,7 | 275,7 | 86 7 | 94 0 | 39 9 |
| | IL-2 + anti-CD1b | 8.701,5 | 2.921,2 | 166.0 | 183.7 | 122 8 |
| with tumor | Control | 287,8 | 7.048,9 | 43 4 | 137.801.5 | 133 4 |
| | IL-2 (250U/ml) | 558,4 | 8.016,0 | 50 5 | 182 374 7 | 376 6 |
| | anti-CD1b ((5µg/ml) | 1000 | 9.745,5 | 67.6 | 250 000 | 1 008 7 |
| | IL-2 + anti-CD1b | 13.859.9 | 11.123.3 | 219.5 | 260 000 | 1 266 0 |

### Abbreviations

- APC: antigen-presenting cell
- PMB: polymyxin B
- CBA: cytokine bead array
- CD: cluster of differentiation
- DC: dendritic cell
- ELISA: enzyme-linked immunosorbent assay
- FSC: forward scatter
- GM-CSF: granulocyte/macrophage-colony-stimulating factor
- HLA-DR: human leukocyte antigen - DR
- HMB-PP: (E)-4-hydroxy-3-methyl-but-2-enyl pyrophosphate
- Hsp: heat shock protein
- i.d.: intradermal
- IFN: interferon
- IL: interleukin
- i.n.: intranodal
- i.v.: intravenous
- KLH: keyhole limpet hemocyanin
- LPS: lipopolysaccharide
- MHC: major histocompatibility complex
- NK: natural killer
- PBS: phosphate buffered saline
- RCC: renal cell carcinoma
- SSC: side scatter
- TCR: T cell receptor
- TGF: transforming growth factor
- Th: T helper
- TNF: tumour necrosis factor

The following embodiments of the present invention are preferred:
1.: Method for providing a preparation of stimulated cytotoxic T cells and NK cells, comprising the following steps:
   - providing a preparation of CD1b-positive (CD1b⁺) antigen presenting cells,
   - stimulating the preparation of CD1b⁺ antigen presenting cells with an anti-CD1b antibody and interleukin 2 (IL-2), and
   - cultivating the stimulated CD1b⁺ antigen presenting cells in a culture medium to obtain stimulated cytotoxic T cells and NK cells.
2.: Method according to embodiment 1, characterised in that a preparation of peripheral blood mononuclear cells (PBMCs) or a subset thereof, especially a preparation of CD56⁺ PBMCs, are provided as preparation of CD1b⁺ antigen presenting cells.
3.: Method according to embodiment 1 or 2, characterised in that cultivation is performed for at least 6, preferably for at least 12, especially for at least 16 hours.
4.: Method according to any one of embodiments 1 to 3, characterised in that the preparation of CD1b⁺ antigen presenting cells is obtained from peripheral blood, bone marrow or umbilical cord blood, especially peripheral blood.
5.: Method according to any one of embodiments 1 to 4, characterised in that the preparation of CD1b⁺ antigen presenting cells contains a cell culture medium.
6.: Method according to any one of embodiments 1 to 5, characterised in that the stimulated cytotoxic T cells and NK cells are washed with an isosmotic T cell washing solution, especially saline and phosphate buffered saline (PBS).
7.: Method according to any one of embodiments 1 to 6, characterised in that the stimulated cytotoxic T cells and NK cells are finished to an injectable pharmaceutical formulation, especially an intradermally, intravenously, intraarterially or subcutaneously injectable formulation or a formulation to be injected into a lymph node.
8.: Method according to any one of embodiments 1 to 7, characterised in that cultivation is performed at least for a duration until T cells and NK cells start to proliferate or produce cytokines.
9.: Method according to any one of embodiments 1 to 8, characterised in that cultivation comprises splitting of the preparation containing cultured stimulated cytotoxic γδ T cells and preferably addition of culture medium and/or further cultivating the preparation.
10.: Method according to any one of embodiments 1 to 9, characterised in that the culture medium is AIM-V, CellGro or RPMI1640 supplemented with 1-5 % autologous human plasma.
11.: Method according to any one of embodiments 1 to 10, characterised in that cultivation is performed for 6 h to 30 d, preferably from 12 h to 20 d, especially from 24 h to 10 d.
12.: Method according to any one of embodiments 1 to 11, characterised in that IL-2 is added at a concentration of 1 to 5000 U/ml, preferably 10 to 1000 U/ml, especially 50 to 500 U/ml.
13.: Method according to any one of embodiments 1 to 12, characterised in that cultivation is performed at 30 to 40°C, preferably at 34 to 38°C, especially at 37°C.
14.: Method according to any one of embodiments 1 to 13, characterised in that the cultivation is performed for a time to increase the number of T cells, especially γδ T cells, by a factor of at least 10, preferably of at least 50, especially at least 100.
15.: Method according to any one of embodiments 1 to 14, characterised in that the anti-CD1b antibody is a CD1b-binding IgG, IgM, IgA, IgD or IgE antibody or a CD1b-binding fragment thereof, especially an Fab, F(ab')₂ or Fv; or an antibody derivative selected from CD1b-binding bi-, tri- or multispecific antibody, disulphide linked Fv, scFv, closed conformation multispecific antibody, disulphide-linked scFv, or diabody.
16.: Preparation of stimulated cytotoxic T cells and NK cells, obtainable by the method according to any one of embodiments 1 to 15.
17.: Preparation according to embodiment 16, characterised in that stimulated T cells and/or NK cells express interferon-γ (IFN-γ) and/or interleukin-17A (IL-17A) and/or interleukin-22 (IL-22).
18.: Preparation according to embodiment 16 or 17, characterised in that the cells have a cytotoxicity of at least 10 % of all tumour cells, preferably at least 50 % of all tumour cells, especially at least 90 % of all tumour cells.
19.: Preparation according to any one of embodiments 16 to 18, characterised in that it contains NK cells, αβ T cells, γδ T cells, or mixtures of such cells.
20.: Preparation according to any one of embodiments 16 to 19, characterised in that it is provided in a pharmaceutically acceptable form, preferably containing an isosmotic lymphocyte washing solution, especially saline or phosphate buffered saline (PBS).
21.: Preparation according to any one of embodiments 16 to 20, characterised in that it contains added active substances, preferably at least one tumour antigen, especially a preparation of lysed tumour cells; at least one cytokine, especially IL-2; or mixtures thereof.
22.: Preparation according to any one of embodiments 16 to 21, characterised in that it contains 1 to 10 x 10⁶ γδ T cells with antigen-presenting capacity or 1 x 10⁶ to 1 x 10⁸ γδ T cells as effector cells per dose.
23.: Preparation according to any one of embodiments 16 to 21, characterised in that it is present in frozen form.
24.: Method for the preparation of a tumour vaccine comprising the following steps:
   - providing a preparation containing stimulated cytotoxic T cells and NK cells according to any one of embodiments 16 to 23,
   - contacting said stimulated cytotoxic T cells and NK cells with tumour cells, especially autologous tumour cells of a cancer patient,
   - cultivating the mixture of stimulated cytotoxic T cells and NK cells and tumour cells to obtain a mixture comprising lysed tumour cells and pro-inflammatory cytokines, and
   - optionally finishing the mixture of lysed tumour cells and pro-inflammatory cytokines to a pharmaceutical preparation.
25.: Method according to embodiment 24, characterised in that the tumour cells are inhibited in growth, preferably by irradiation, chemotherapy drugs or combinations thereof.
26.: Method according to embodiments 24 or 25, characterised in that the mixture comprising lysed tumour cells and pro-inflammatory cytokines is contacted for loading and/or maturation of dendritic cells (DCs) in vitro to obtain a DC-based tumour vaccine.
27.: Method for in vitro stimulation of white blood cells characterised by the following steps:
   - providing a preparation of white blood cells,
   - contacting the preparation of white blood cells with an anti-CD1b antibody and interleukin 2 (IL-2), and
   - cultivating the stimulated white blood cells, and
   - measuring at least one phenotypic property of the stimulated and cultivated white blood cells.
28.: Method according to embodiment 27, characterised in that the phenotypic property is expression level of one or more cytokines, preferably IFN-γ and/or IL-17A and IL-22, or cell expansion.
29.: Method according to embodiment 27 or 28, characterised in that the preparation of white blood cells was obtained from a patient whose immune status has to be monitored and the method is used for monitoring the immune status of this patient.
30.: Method according to embodiment 29, characterised in that
   - at least two preparations of white blood cells were obtained from a patient at different points in time,
   - the same one or more phenotypic properties are measured in the at least two preparations of white blood cells, and
   - the measured phenotypic properties of the at least two preparations are compared with each other to monitor the immune status of this patient.
31.: Kit for stimulation of white blood cells comprising
   - an anti-CD1b-antibody, and
   - IL-2.
32.: A pharmaceutical composition comprising an anti-CD1b-antibody, preferably for use in immune stimulation, especially for the treatment of infectious diseases or tumour diseases.
33.: Pharmaceutical composition according to embodiment 32, characterised in that it comprises a vaccine antigen, especially a vaccine antigen for vaccination against an infectious disease or a tumour vaccination antigen.

## Claims

1. : Method for providing a preparation of stimulated cytotoxic T cells and NK cells, comprising the following steps:
- providing a preparation of CD1b-positive (CD1b⁺) antigen presenting cells,
- stimulating the preparation of CD1b⁺ antigen presenting cells with an anti-CD1b antibody and interleukin 2 (IL-2), and
- cultivating the stimulated CD1b⁺ antigen presenting cells in a culture medium to obtain stimulated cytotoxic T cells and NK cells.

2. : Method according to claim 1, **characterised in that** a preparation of peripheral blood mononuclear cells (PBMCs) or a subset thereof, especially a preparation of CD56⁺ PBMCs, are provided as preparation of CD1b⁺ antigen presenting cells.

3. : Method according to claim 1 or 2, **characterised in that** the stimulated cytotoxic T cells and NK cells are finished to an injectable pharmaceutical formulation, especially an intradermally, intravenously, intraarterially or subcutaneously injectable formulation or a formulation to be injected into a lymph node.

4. : Method according to any one of claims 1 to 3, **characterised in that** cultivation is performed for 6 h to 30 d, preferably from 12 h to 20 d, especially from 24 h to 10 d.

5. : Method according to any one of claims 1 to 4, **characterised in that** IL-2 is added at a concentration of 1 to 5000 U/ml, preferably 10 to 1000 U/ml, especially 50 to 500 U/ml.

6. : Method according to any one of claims 1 to 5, **characterised in that** cultivation is performed at 30 to 40°C, preferably at 34 to 38°C, especially at 37°C.

7. : Preparation of stimulated cytotoxic T cells and NK cells, obtainable by the method according to any one of claims 1 to 6.

8. : Preparation according to claim 7, **characterised in that** stimulated T cells and/or NK cells express IFN-γ and/or interleukin-17A (IL-17A) and/or interleukin-22 (IL-22).

9. : Preparation according to claim 7 or 8, **characterised in that** the cells have a cytotoxicity of at least 10 % of all tumour cells, preferably at least 50 % of all tumour cells, especially at least 90 % of all tumour cells.

10. : Preparation according to any one of claims 7 to 9, **characterised in that** it contains NK cells, αβ T cells, γδ T cells, or mixtures of such cells.

11. : Preparation according to any one of claims 7 to 10, **characterised in that** it contains added active substances, preferably at least one tumour antigen, especially a preparation of lysed tumour cells; at least one cytokine, especially IL-2; a microbiological antigen or mixtures thereof.

12. : Method for the preparation of a tumour vaccine comprising the following steps:
- providing a preparation containing stimulated cytotoxic T cells and NK cells according to any one of claims 7 to 11,
- contacting said stimulated cytotoxic T cells and NK cells with tumour cells, especially autologous tumour cells of a cancer patient,
- cultivating the mixture of stimulated cytotoxic T cells and NK cells and tumour cells to obtain a mixture comprising lysed tumour cells and pro-inflammatory cytokines, and
- optionally finishing the mixture of lysed tumour cells and pro-inflammatory cytokines to a pharmaceutical preparation.

13. : Method for in vitro stimulation of white blood cells **characterised by** the following steps:
- providing a preparation of white blood cells,
- contacting the preparation of white blood cells with an anti-CD1b antibody and interleukin 2 (IL-2), and
- cultivating the stimulated white blood cells, and
- measuring at least one phenotypic property of the stimulated and cultivated white blood cells.

14. : Kit for stimulation of white blood cells comprising
- an anti-CD1b-antibody, and
- IL-2.

15. : A pharmaceutical composition comprising an anti-CD1b-antibody, preferably for use in immune stimulation, especially for the treatment of infectious diseases or tumour diseases.
